# EUROPEAN PATENT SPECIFICATION

(11) **EP 1 660 032 B1**
(45) Date of publication and mention of the grant of the patent: **02.07.2008**
(21) Application number: 04764153.5
(22) Date of filing: 02.08.2004
(51) Int. Cl.: A61K 8/49, A61Q 5/10

(54) **COMPOSITION FOR DYEING HUMAN KERATIN FIBRES, COMPRISING A 1H-PYRAZOLYL-ETHENYL-INDOLIUM DERIVATIVE**
ZUSAMMENSETZUNG ZUM FÄRBEN VON MENSCHLICHEN KERATINFASERN MIT EINEM 1H-PYRAZOLYL-ETHENYL-INDOLIUM DERIVAT
COMPOSITIONS DE COLORATION DE FIBRES KERATINIQUES HUMAINES CONTENANT UN DERIVE DE 1H-PYRAZOLYL-ETHENYL-INDOLE

(30) Priority: 05.08.2003 FR 0309659; 29.09.2003 US 506514 P
(43) Date of publication of application: 31.05.2006
(73) Proprietor: L'ORÉAL, 75008 Paris (FR)
(72) Inventor: GREAVES, Andrew, 77144 Montevrain (FR)
(74) Representative: Fevrier, Murielle Françoise E.
(86) International application number: PCT/EP2004/009161
(87) International publication number: WO 2005/013929

(56) References cited:
- DE-A- 2 345 462
- GB-A- 1 301 492

## Description

The invention relates to a composition for dyeing human keratin fibres, comprising, in a suitable medium, a 1H-pyrazolyl-ethenyl-indolium derivative, and also to the dyeing process using this composition.

It is known practice to dye keratin fibres, and in particular human hair, with dye compositions containing oxidation dye precursors, which are generally known as oxidation bases, such as ortho- or para-phenylenediamines, ortho- or para-aminophenols and heterocyclic compounds. These oxidation bases are colourless or weakly coloured compounds, which, when combined with oxidizing products, may give rise to coloured compounds via a process of oxidative condensation. It is also known that the shades obtained may be varied by combining these oxidation bases with couplers or coloration modifiers, the latter being chosen especially from aromatic meta-diamines, meta-aminophenols, meta-diphenols and certain heterocyclic compounds such as indole compounds. The oxidation dyeing process consists in applying to the keratin fibres bases or a mixture of bases and couplers with aqueous hydrogen peroxide solution as oxidizing agent, leaving the mixture to act and then rinsing the fibres.

The variety of molecules used as oxidation bases and couplers allows a wide range of colours to be obtained.

The colorations resulting therefrom are permanent, strong and resistant to external agents, especially to light, bad weather, washing, perspiration and rubbing. However, oxidation dyeing requires the use of an oxidizing agent and also pH conditions which are such that the keratin fibres thus treated are often impaired after one or more dyeing operations.

It is also known practice to dye human keratin fibres, and in particular the hair, by direct dyeing. The process conventionally used in direct dyeing consists in applying to the keratin fibres direct dyes, which are coloured and colouring molecules that have affinity for the fibres, leaving the mixture to act and then rinsing the fibres.

The direct dyes that are generally used are dyes of the nitrobenzene type, anthraquinone dyes, nitropyridines or dyes of the azo, xanthene, acridine, azine or triarylmethane type.

Although this type of dyeing respects the integrity of the keratin fibre, the colorations obtained using these direct dyes are temporary or semi-permanent since the nature of the interactions that bind the direct dyes to the keratin fibre, and their desorption from the surface and/or from the core of the fibre are responsible for their weak dyeing power and their poor fastness with respect to washing or perspiration.

The aim of the present invention is to provide novel compositions for the direct dyeing of human keratin fibres, which do not have the drawbacks of the compositions of the prior art. In particular, the aim of the invention is to provide novel compositions for dyeing human keratin fibres, which show strong affinity for these keratin fibres, in particular the hair, in order to increase the resistance of the coloration to external agents, such as resistance to shampooing, to light or to sweat, while at the same time respecting the integrity of the keratin fibres.

This aim is achieved with the present invention, one subject of which is a dye composition comprising, in a suitable cosmetic medium, a 1H-pyrazolyl-ethenyl-indolium derivative of formula (I) below: 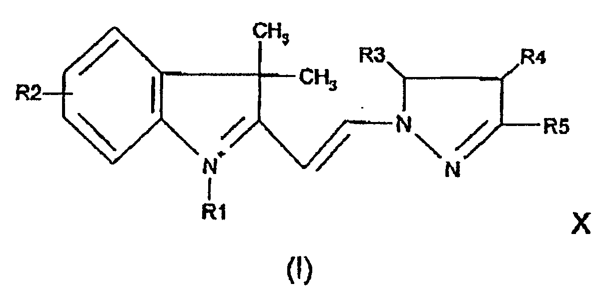 in which
R1 represents a linear or branched C1-C4 alkyl radical or a C1-C4 aralkyl radical,
R2 represents a hydrogen atom, a C1-C4 alkyl radical, a C1-C4 alkoxy radical, a halogen atom or a nitro group;
R3 and R4, which may be identical or different, represent a hydrogen atom, a C1-C4 alkyl radical, an aryl radical which may be substituted with one or more radicals R chosen from a halogen atom, a hydroxyl radical, a C1-C4 alkyl radical, a C1-C4 alkoxy radical, a carboxyl radical and a trifluoromethyl radical,
R5 represents a hydrogen atom, a C1-C4 alkyl radical or an aryl radical that may be substituted with one or more radicals R, and
X is a counterion, for example a chloride, bromide or methyl sulfate ion, and comprising a surfactant as defined in claim 1*.*

A subject of the invention is also a dyeing process using a composition comprising the derivative of formula (I).

Another subject of the invention is the use of the 1H-pyrazolyl-ethenyl-indolium derivative of formula (I) for dyeing human keratin fibres such as the hair.

Such a composition makes it possible to obtain direct colourations that are particularly resistant to external agents, in particular to shampoo.

In formula (I) above, R1 may be a linear or branched methyl, ethyl, propyl, butyl or pentyl radical, or a benzyl radical. R1 preferably represents a C1-C4 alkyl radical.

R2 may represent a hydrogen atom, a linear or branched methyl, ethyl or propyl radical, a methoxy, ethoxy or propyloxy radical, a chlorine or bromine atom, or a nitro radical. R2 preferably represents a hydrogen atom or an alkyl or alkoxy radical, in particular a hydrogen atom or a methyl or methoxy radical.

By way of example for R3 and R4, mention may be made of a hydrogen atom, a methyl, ethyl, propyl or butyl radical, or a phenyl radical, which may be substituted. Preferably, R3 and R4 independently represent a hydrogen atom or an alkyl or phenyl radical. According to one particular embodiment, R3 represents a hydrogen atom, a methyl radical or a phenyl radical, which may be substituted with one or more hydroxyl, alkoxy, methyl or chloro radicals and R4 represents a hydrogen atom or a phenyl radical, which may be substituted with one or more hydroxyl, alkoxy, methyl or chloro radicals.

In formula (I), R5 may be a hydrogen atom, a methyl, ethyl, propyl or butyl radical, or a phenyl radical, which may be substituted. According to one particular embodiment, R5 represents a hydrogen atom or an alkyl or phenyl radical. Preferably, R5 represents a hydrogen atom, a methyl radical or a phenyl radical, which may be substituted with one or more hydroxyl, alkoxy, methyl or chloro radicals.

By way of example, mention may be made of the derivatives of formula (I) below:

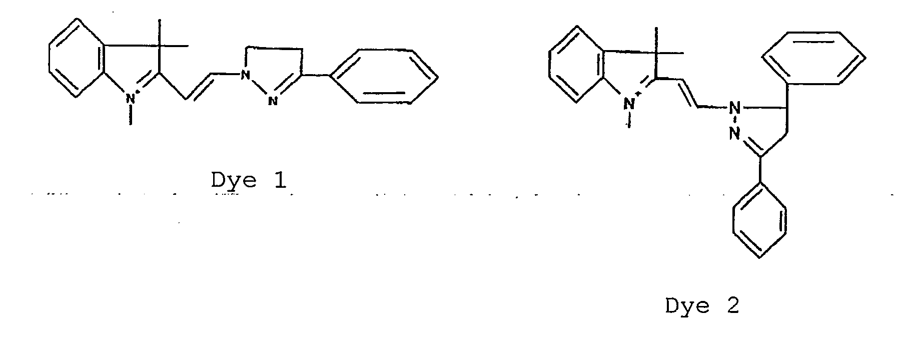

According to one particularly preferred embodiment, R1 represents a methyl radical, R2 represents a hydrogen atom, R4 represents a methyl radical and, when R5 represents 4-hydroxyphenyl, then R3 represents a methyl radical, when R5 represents a 4-methoxyphenyl radical, then R3 represents a hydrogen atom, when R5 represents a 2-hydroxyphenyl radical, then R3 represents a phenyl radical, or when R5 represents a 4-chlorophenyl radical, then R3 represents a 2-hydroxyphenyl radical.

The composition of the present invention generally comprises between 0.01% and 20% by weight of 1H-pyrazolyl-ethenyl-indolium derivatives and preferably between 0.1% and 5% relative to the total weight of the composition.

The 1H-pyrazolyl-ethenyl-indolium derivatives that are useful for the present invention may be synthesized via methods that are well known to those skilled in the art, in particular the methods described in documents DE 2 345 462 and GB 1 301 492.

The composition of the present invention may contain one or more other dyes, for example direct dyes, oxidation bases or couplers.

The direct dyes may be chosen especially from nitrobenzene dyes, azo direct dyes and methine direct dyes. These direct dyes may be of nonionic, anionic or cationic nature. The direct dyes that may be used according to the invention are preferably chosen from neutral, acidic or cationic nitrobenzene direct dyes, neutral, acidic or cationic azo direct dyes, quinone direct dyes and in particular neutral, acidic or cationic anthraquinones, azine direct dyes, triarylmethane direct dyes, indoamine direct dyes and natural direct dyes.

Among the nitrobenzene dyes that may be mentioned are the following red or orange compounds: 1-hydroxy-3-nitro-4-N-(y-hydroxypropyl)aminobenzene, N-(β-hydroxyethyl)amino-3-nitro-4-aminobenzene, 1-amino-3-methyl-4-N-(β-hydroxyethyl)amino-6-nitrobenzene, 1-hydroxy-3-nitro-4-N-(β-hydroxyethyl)-aminobenzene, 1,4-diamino-2-nitrobenzene, 1-amino-2-nitro-4-methylaminobenzene, N-(β-hydroxyethyl)-2-nitroparaphenylenediamine, 1-amino-2-nitro-4-(β-hydroxyethyl)amino-5-chlorobenzene, 2-nitro-4-aminodiphenylamine, 1-amino-3-nitro-6-hydroxybenzene, 1-(β-aminoethyl)amino-2-nitro-4-(β-hydroxyethyloxy)-benzene, 1-(β,γ-dihydroxypropyl)oxy-3-nitro-4-(β-hydroxyethyl)aminobenzene, 1-hydroxy-3-nitro-4-aminobenzene, 1-hydroxy-2-amino-4,6-dinitrobenzene, 1-methoxy-3-nitro-4-(β-hydroxyethyl)aminobenzene, 2-nitro-4'-hydroxydiphenylamine, 1-amino-2-nitro-4-hydroxy-5-methylbenzene, alone or as mixtures.

As regards the nitrobenzene direct dyes, use may be made of dyes of yellow and green-yellow type, for instance 1-β-hydroxyethyloxy-3-methylamino-4-nitrobenzene, 1-methylamino-2-nitro-5-(β,γ-dihydroxypropyl)oxybenzene, 1-(β-hydroxyethyl)amino-2-methoxy-4-nitrobenzene, 1-(β-aminoethyl)amino-2-nitro-5-methoxybenzene, 1,3-bis(β-hydroxyethyl)amino-4-nitro-6-chlorobenzene, 1-amino-2-nitro-6-methylbenzene, 1-(β-hydroxyethyl)amino-2-hydroxy-4-nitrobenzene, N-(β-hydroxyethyl)-2-nitro-4-trifluoromethylaniline, 4-(β-hydroxyethyl)amino-3-nitrobenzenesulphonic acid, 4-ethylamino-3-nitrobenzoic acid, 4-β-hydroxyethyl)-amino-3-nitrochlorobenzene, 4-(β-hydroxyethyl)amino-3-nitromethylbenzene, 4-(β,γ-dihydroxypropyl)amino-3-nitrotrifluoromethylbenzene, 1-(β-ureidoethyl)amino-4-nitrobenzene, 1,3-diamino-4-nitrobenzene, 1-hydroxy-2-amino-5-nitrobenzene, 1-amino-2-[tris(hydroxymethyl)-methyl]amino-5-nitrobenzene, 1-(β-hydroxyethyl)amino-2-nitrobenzene and 4-(β-hydroxyethyl)amino-3-nitrobenzamide.

It may also be envisaged to use blue or violet nitrobenzene dyes, for instance, inter alia, 1-(β-hydroxyethyl)amino-4-N,N-bis(β-hydroxyethyl)amino-2-nitrobenzene, 1-(y-hydroxypropyl)amino-4-N,N-bis-(β-hydroxyethyl)amino-2-nitrobenzene, 1-(β-hydroxyethyl)amino-4-(N-methyl, N-β-hydroxyethyl)amino-2-nitrobenzene, 1-(β-hydroxyethyl)amino-4-(N-ethyl, N-β-hydroxyethyl) amino-2-nitrobenzene, 1-(β,γ-dihydroxypropyl)amino-4-(N-ethyl, N-β-hydroxyethyl)amino-2-nitrobenzene, the 2-nitro-para-phenylenediamines of the following formula: 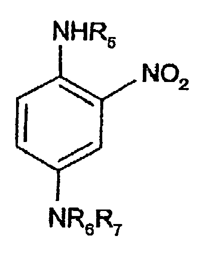 in which:
- R₆ represents a C₁-C₄ alkyl radical or a β-hydroxyethyl, β-hydroxypropyl or γ-hydroxypropyl radical;
- R₅ and R₇, which may be identical or different, represent a β-hydroxyethyl, β-hydroxypropyl, γ-hydroxypropyl or β,γ-dihydroxypropyl radical, at least one of the radicals R₆, R₇ or R₅ representing a γ-hydroxypropyl radical and R₆ and R₇ not being able simultaneously to denote a β-hydroxyethyl radical when R₆ is a γ-hydroxypropyl radical, such as those described in French patent FR 2 692 572.

It is recalled that azo dyes are compounds comprising in their structure at least one -N=N-sequence not included in a ring; methine dyes are compounds comprising in their structure at least one -C=C- sequence not included in a ring; azomethine dyes are compounds comprising in their structure at least one -C=N- sequence not included in a ring.

The triarylmethane-based dyes comprise in their structure at least one sequence below: 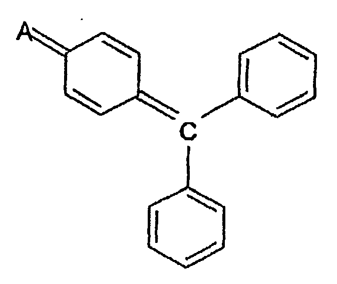 A denoting an oxygen or nitrogen atom.

The xanthene dyes comprise in their structure at least one sequence of formula:

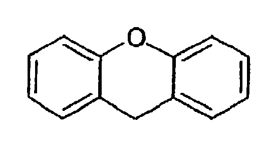

The phenanthridine dyes comprise in their structure at least one sequence of formula:

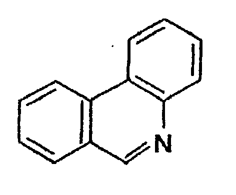

The phthalocyanin dyes comprise in their structure at least one sequence of formula:

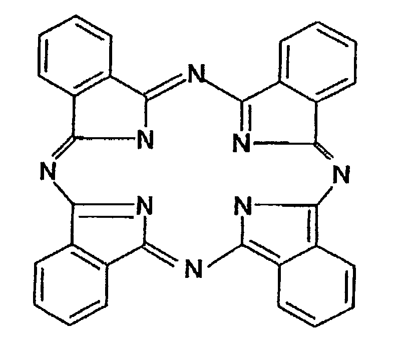

The phenothiazine dyes comprise in their structure at least one sequence below:

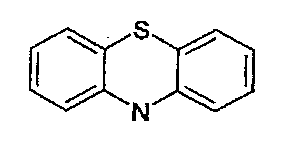

The direct dyes may moreover be chosen from basic dyes like those listed in the Color Index, 3rd edition, especially under the names "Basic Brown 16", "Basic Brown 17", "Basic Yellow 57", "Basic Red 76", "Basic Violet 10", "Basic Blue 26" and "Basic Blue 99"; or from the acidic direct dyes listed in the Color Index, 3rd edition, under the names "Acid Orange 7", "Acid Orange 24", "Acid Yellow 36", "Acid Red 33", "Acid Red 184", "Acid Black 2", "Acid Violet 43", and "Acid Blue 62", or cationic direct dyes such as those described in patent applications WO 95/01772, WO 95/15144 and EP 714 954, the content of which forms an integral part of the present invention.

When they are present, the direct dye(s) preferably represent(s) from 0.0005% to 12% by weight approximately relative to the total weight of the composition and even more preferably from 0.005% to 6% by weight approximately relative to this weight.

The oxidation bases that may be used in the composition of the present invention are the oxidation bases conventionally used in oxidation dyeing. By way of example, these oxidation bases are chosen from, for example para-phenylenediamines, bis(phenyl)alkylenediamines, para-aminophenols, bis-para-aminophenols, ortho-aminophenols and heterocyclic bases, and the addition salts thereof.

Among the para-phenylenediamines that may more particularly be mentioned, are para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, N,N-diethylamino-3-methyl-para-phenylenediamine, N,N-bis(β-hydroxyethyl)-para-phenylenediamine, 4N,N-bis(β-hydroxyethyl)amino-2-methylaniline, N,N-bis(β-hydroxyethyl)amino-2-chloro-para-phenylenediamine, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetyl-aminoethyloxy-para-phenylenediamine and N-(β-methoxyethyl)-para-phenylenediamine, and the addition salts thereof with an acid.

Among the para-phenylenediamines mentioned above, para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof with an acid, are very particularly preferred.

Among the bis(phenyl)alkylenediamines that may more particularly be mentioned, for example, are N,N'-bis(β-hydroxyethyl)-N,N'-bis(4'-aminophenyl)-1,3-diaminopropanol, N,N'-bis(β-hydroxyethyl)-N,N'-bis(4'-aminophenyl) ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis-(4'-amino-3'-methylphenyl)ethylenediamine and 1,8-bis-(2,5-diaminophenoxy)-3,5-dioxaoctane, and the addition salts thereof with an acid.

Among the para-aminophenols that may more particularly be mentioned, for example, are para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols that may more particularly be mentioned, for example, are 2-aminophenol, 2-amino-5-methylphenol, 2-amino-6-methylphenol and 5-acetamido-2-aminophenol, and the addition salts thereof with an acid.

Among the heterocyclic bases that may more particularly be mentioned, for example, are pyridine derivatives, pyrimidine derivatives and pyrazole derivatives.

Among the pyridine derivatives that may more particularly be mentioned are the compounds described, for example, in patents GB 1 026 978 and GB 1 153 196, such as 2,5-diaminopyridine, 2-(4-methoxyphenyl)amino-3-aminopyridine, 2,3-diamino-6-methoxypyridine, 2-(β-methoxyethyl)amino-3-amino-6-methoxypyridine and 3,4-diaminopyridine, and the addition salts thereof with an acid.

Among the pyrimidine derivatives that may more particularly be mentioned are the compounds described, for example, in patents DE 2 359 399; JP 88-169 571; JP 05-163 124; EP 0 770 375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine and 2,5,6-triaminopyrimidine, and pyrazolopyrimidine derivatives such as those mentioned in patent application FR-A-2 750 048 and among which mention may be made of pyrazolo[1,5-a]-pyrimidine-3,7-diamine; 2,5-dimethylpyrazolo[1,5-a]-pyrimidine-3,7-diamine; pyrazolo[1,5-a]pyrimidine-3,5-diamine; 2,7-dimethylpyrazolo[1,5-a]pyrimidine-3,5-diamine; 3-aminopyrazolo[1,5-a]pyrimidin-7-ol; 3-aminopyrazolo[1,5-a]pyrimidin-5-ol; 2-(3-amino-pyrazolo[1,5-a]pyrimidin-7-ylamino)ethanol, 2-(7-amino-pyrazolo[1,5-a]pyrimidin-3-ylamino)ethanol, 2-[(3-aminopyrazolo[1,5-a]pyrimidin-7-yl)(2-hydroxyethyl)amino]ethanol, 2-[(7-aminopyrazolo[1,5-a]-pyrimidin-3-yl)(2-hydroxyethyl)amino]ethanol, 5,6-dimethylpyrazolo[1,5-a]pyrimidine-3,7-diamine, 2,6-dimethylpyrazolo[1,5-a]pyrimidine-3,7-diamine, 2,5,N7,N7-tetramethylpyrazolo[1,5-a]pyrimidine-3,7-diamine and 3-amino-5-methyl-7-imidazolylpropyl-aminopyrazolo[1,5-a]pyrimidine, and the addition salts thereof with an acid and the tautomeric forms thereof, when a tautomeric equilibrium exists.

Among the pyrazole derivatives that may more particularly be mentioned are the compounds described in patents DE 3 843 892 and DE 4 133 957 and patent applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the addition salts thereof with an acid.

When they are used, these oxidation bases preferably represent from 0.0005% to 12% by weight approximately relative to the total weight of the dye composition, and even more preferably from 0.005% to 6% by weight approximately relative to this weight.

The couplers may be chosen from the couplers conventionally used in oxidation dyeing, and among which mention may be made especially of meta-phenylenediamines, meta-aminophenols, meta-diphenols, naphthols and heterocyclic couplers, for instance indole derivatives, indoline derivatives, pyridine derivatives, indazole derivatives, pyrazolo[1,5-b]-1,2,4-triazole derivatives, pyrazolo[3,2-c]-1,2,4-triazole derivatives, benzimidazole derivatives, benzothiazole derivatives, benzoxazole derivatives, 1,3-benzodioxole derivatives and pyrazolones, and the addition salts thereof with an acid.

These couplers are more particularly chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy) benzene, 2-amino-4-(β-hydroxyethylamino)-1-methoxybenzene, 1,3-diaminobenzene, 1,3-bis(2,4-diaminophenoxy)propane, sesamol, α-naphthol, 2-methyl-1-naphthol, 6-hydroxyindole, 4-hydroxyindole, 4-hydroxy-N-methylindole, 6-hydroxyindoline, 6-hydroxybenzomorpholine, 3,5-diamino-2,6-dimethoxy-pyridine, 1-N(β-hydroxyethyl)amino-3,4-methylenedioxybenzene, 2,6-bis(β-hydroxyethyleneamino)toluene, 2,6-dihydroxy-4-methylpyridine, 1H-3-methylpyrazol-5-one, 1-phenyl-3-methylpyrazol-5-one, and the addition salts thereof with an acid.

When they are used, the coupler(s) preferably represent(s) from 0.0001% to 10% by weight approximately relative to the total weight of the dye composition, and even more preferably from 0.005% to 5% by weight approximately relative to this weight.

The medium that is suitable for dyeing, also known as the dye support, is generally a cosmetic medium consisting of water or of a mixture of water and of at least one organic solvent to dissolve the compounds that would not be sufficiently water-soluble. Examples of organic solvents that may be mentioned include C₁-C₄ lower alkanols, such as ethanol and isopropanol; polyols and polyol ethers, for instance 2-butoxyethanol, propylene glycol, propylene glycol monomethyl ether, diethylene glycol monoethyl ether and monomethyl ether, and also aromatic alcohols, for instance benzyl alcohol or phenoxyethanol, and mixtures thereof.

The solvents are preferably present in proportions preferably of between 1% and 40% by weight approximately relative to the total weight of the dye composition, and even more preferably between 5% and 30% by weight approximately.

The dye composition in accordance with the invention may also contain various adjuvants conventionally used in compositions for dyeing the hair, such as anionic, cationic, nonionic, amphoteric or zwitterionic surfactants, or mixtures thereof.

By way of example of anionic surfactants that can be used, alone or as mixtures, mention may be made in particular of salts (in particular alkali metal salts, especially sodium salts, ammonium salts, amine salts, amino alcohol salts or magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, α-olefin sulphonates, paraffin sulphonates; (C₆-C₂₄)alkyl sulphosuccinates, (C₆-C₂₄)alkyl ether sulphosuccinates, (C₆-C₂₄) alkylamide sulphosuccinates; (C₆-C₂₄)alkyl sulphoacetates; (C₆-C₂₄)acyl sarcosinates and (C₆-C₂₄)-acyl glutamates. It is also possible to use (C₆-C₂₄) alkylpolyglycoside carboxylic esters such as alkylglucoside citrates, alkylpolyglycoside tartrates and alkylpolyglycoside sulphosuccinates, alkylsulphosuccinamates; acyl isethionates and N-acyl taurates, the alkyl or acyl radical of all of these different compounds preferably containing from 12 to 20 carbon atoms and the aryl radical preferably denoting a phenyl or benzyl group. Among the anionic surfactants which can also be used, mention may also be made of fatty acid salts such as oleic, ricinoleic, palmitic and stearic acid salts, coconut oil acids or hydrogenated coconut oil acid; acyl lactylates in which the acyl radical contains more particularly 8 to 20 carbon atoms. It is also possible to use alkyl D-galactoside uronic acids and their salts, polyoxyalkylenated (C₆-C₂₄) alkyl ether carboxylic acids, polyoxyalkylenated (C₆-C₂₄)alkylaryl ether carboxylic acids, polyoxyalkylenated (C₆-C₂₄)alkylamido ether carboxylic acids and their salts, in particular those containing from 2 to 50 alkylene oxide groups, in particular ethylene oxide groups, and mixtures thereof. The nonionic surfactants are, themselves also, compounds that are well known per se (see in particular in this respect "Handbook of Surfactants" by M.R. Porter, published by Blackie & Son (Glasgow and London), 1991, pp. 116-178). They can be chosen in particular from polyethoxylated and/or polypropoxylated, alkylphenols, alpha-diols or alcohols, having a fatty chain containing, for example, 8 to 18 carbon atoms, it being possible for the number of ethylene oxide and/or propylene oxide groups to range in particular from 2 to 50. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide, polyglycerolated fatty amides containing on average 1 to 5, and in particular 1.5 to 4, glycerol groups; polyethoxylated fatty amines preferably having 2 to 30 mol of ethylene oxide; oxyethylenated fatty acid esters of sorbitan having from 2 to 30 mol of ethylene oxide; fatty acid esters of sucrose, fatty acid esters of polyethylene glycol, alkylpolyglycosides, N-alkylglucamine derivatives, and amine oxides such as (C₁₀-C₁₄)alkylamine oxides or N-acylaminopropylmorpholine oxides.

The amphoteric or zwitterionic surfactants can be, in particular chosen from aliphatic secondary or tertiary amine derivatives in which the aliphatic radical is a linear or branched chain containing 8 to 18 carbon atoms and containing at least one water-solubilizing anionic group (for example carboxylate, sulphonate, sulphate, phosphate or phosphonate); mention may also be made of (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines.

Among the amine derivatives, mention may be made of the products sold under the name Miranol, as described in US patents 2 528 378 and 2 781 354 and classified in the CTFA dictionary, 3rd edition, 1982, under the names Amphocarboxyglycinates and Amphocarboxypropionates, with the respective structures:

R₂-CONHCH₂CH₂-N(R₃)(R₄)(CH₂COO⁻)

in which: R₂ denotes an alkyl radical of an acid R₂-COOH present in hydrolysed coconut oil, a heptyl, nonyl or undecyl radical, R₃ denotes a beta-hydroxyethyl group and R₄ denotes a carboxymethyl group; and
R₂'-CONHCH₂CH₂-N (B) (C)
in which:
B represents -CH₂CH₂OX', C represents -(CH₂)_{z}-Y', with z = 1 or 2,
X' denotes the -CH₂CH₂-COOH group or a hydrogen atom,
Y' denotes -COOH or the -CH₂-CHOH-SO₃H radical,
R₂' denotes an alkyl radical of an acid R₉-COOH present in coconut oil or in hydrolysed linseed oil, an alkyl radical, in particular a C₇, C₉, C₁₁ or C₁₃ alkyl radical, a C₁₇ alkyl radical and its iso form, or an unsaturated C₁₇ radical.

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

By way of example, mention may be made of the cocoamphodiacetate sold under the trade name Miranol^{®} C2M concentrate by the company Rhodia Chimie.

Among the cationic surfactants, mention may be made in particular of: salts of optionally polyoxyalkylenated primary, secondary or tertiary fatty amines; quaternary ammonium salts such as tetraalkylammonium, alkylamidoalkyltrialkylammonium, trialkylbenzylammonium, trialkylhydroxyalkylammonium or alkylpyridinium chlorides or bromides; imidazoline derivatives; or amine oxides of cationic nature.

The above surfactants are generally present in an amount for each of between 0.01% and 20% by weight relative to the weight of the composition.

The dye composition in accordance with the invention may also contain various adjuvants conventionally used in compositions for dyeing the hair, such as anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrating agents, sequestering agents, fragrances, buffers, dispersants, conditioners, for instance volatile or non-volatile, modified or unmodified silicones, film-forming agents, ceramides, preserving agents and opacifiers.

The above adjuvants are generally present in an amount for each of between 0.01% and 20% by weight relative to the weight of the composition.

Needless to say, a person skilled in the art will take care in selecting this or these optional additional compound(s) such that the advantageous properties intrinsically associated with the oxidation dye composition in accordance with the invention are not, or are not substantially, adversely affected by the envisaged addition(s).

The pH of the dye composition in accordance with the invention is generally between 3 and 12 approximately and preferably between 5 and 11 approximately. It may be adjusted to the desired value using acidifying or basifying agents usually used in the dyeing of keratin fibres, or alternatively using standard buffer systems.

Among the acidifying agents that may be mentioned, for example, are mineral or organic acids, for instance hydrochloric acid, orthophosphoric acid, sulphuric acid, carboxylic acids, for instance acetic acid, tartaric acid, citric acid and lactic acid, and sulphonic acids.

Among the basifying agents that may be mentioned, for example, are aqueous ammonia, alkaline carbonates, alkanolamines such as mono-, di- and triethanolamine, and derivatives thereof, sodium hydroxide, potassium hydroxide and the compounds of formula (II) below: 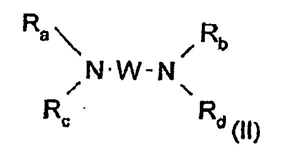 in which W is a propylene residue optionally substituted with a hydroxyl group or a C₁-C₄ alkyl radical; Rₐ, R_{b}, R_{c} and R_{d}, which may be identical or different, represent a hydrogen atom or a C₁-C₄ alkyl or C₁-C₄ hydroxyalkyl radical.

The composition may comprise at least one oxidizing agent. It is chosen more particularly from the oxidizing agents conventionally used in the field, for instance hydrogen peroxide, urea peroxide, alkali metal bromates, persalts such as perborates and persulphates, peracids and oxidase enzymes, among which mention may be made of peroxidases, 2-electron oxidoreductases such as uricases, and 4-electron oxygenases, for instance laccases. Hydrogen peroxide is particularly preferred.

The dye composition according to the invention may be in various forms, such as in the form of liquids, creams or gels, or in any other form that is suitable for dyeing keratin fibres and especially human hair.

The process of the invention is a process for dyeing keratin fibres in which the composition of claim 15 is applied to the keratin fibres for a time that is sufficient to obtain the desired colouration.

The leave-in time for the composition of the invention is generally between 3 and 50 minutes approximately and preferably 5 to 30 minutes approximately.

A subject of the invention is also the use of the 1H-pyrazolyl-ethenyl-indolium derivative for dyeing human keratin fibres such as the hair.

The examples that follow serve to illustrate the invention without, however, being limiting in nature.

### EXAMPLES

5 × 10⁻⁴ mol% of dye of formula DYE 1 is dissolved in a buffer solution at pH 9 (2 g of ammonium acetate in 40 ml of water adjusted to pH 9 by adding concentrated aqueous ammonia, made up to 100 ml with demineralized water). A lock of grey hair is immersed in the solution containing the dye (ratio of the amount of solution:amount of hair = 10:1).

After 20 minutes, the hair is rinsed with demineralized water. The hair is thus dyed yellow.

The same process is performed with the dye DYE 2. The hair is thus dyed yellow, as previously.

## Claims

1. Composition for dyeing human keratin fibres, comprising, in a suitable cosmetic medium, a 1H-pyrazolyl-ethenyl-indolium derivative of formula (I) below: 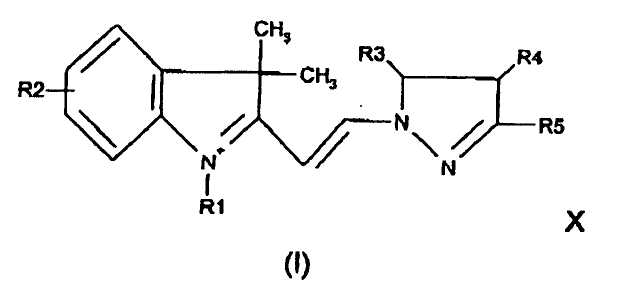 in which
● R1 represents a linear or branched C1-C4 alkyl radical or a C1-C4 aralkyl radical,
● R2 represents a hydrogen atom, a C1-C4 alkyl radical, a C1-C4 alkoxy radical, a halogen atom or a nitro group;
● R3 and R4, which may be identical or different, represent a hydrogen atom, a C1-C4 alkyl radical, an aryl radical which may be substituted with one radical R chosen from a halogen atom, a hydroxyl radical, a C1-C4 alkyl radical, a C1-C4 alkoxy radical, a carboxyl radical and a trifluoromethyl radical,
● R5 represents a hydrogen atom, a C1-C4 alkyl radical or an aryl radical that may be substituted with one radical R, and
● X is a counterion,
and comprising at least one anionic, cationic, nonionic, amphoteric or zwitterionic surfactant, or mixtures thereof.

2. Composition according to Claim 1, in which R1 represents a C1-C4 alkyl radical.

3. Composition according to Claim 1, in which R2 represents a hydrogen atom or an alkyl or alkoxy radical.

4. Composition according to Claim 1 or 2, in which R2 represents a hydrogen atom or a methyl or methoxy radical.

5. Composition according to any one of the preceding claims, in which R3 and R4 represent a hydrogen atom or an alkyl or phenyl radical.

6. Composition according to Claim 4, in which R3 represents a hydrogen atom, a methyl radical or a phenyl radical, which may be substituted with one or more hydroxyl, alkoxy, methyl or chloro radicals.

7. Composition according to Claim 4, in which R4 represents a hydrogen atom or a methyl or phenyl radical, which may be substituted with one or more hydroxyl, alkoxy, methyl or chloro radicals.

8. Composition according to any one of the preceding claims, in which R5 represents a hydrogen atom, an alkyl radical or a phenyl radical, which may be substituted with one or more hydroxyl, alkoxy, methyl or chloro radicals.

9. Composition according to any one of the preceding claims, in which R1 represents a methyl radical, R2 represents a hydrogen atom, R4 represents a methyl radical and, when R5 represents 4-hydroxyphenyl, then R3 represents a methyl radical, when R5 represents a 4-methoxyphenyl radical, then R3 represents a hydrogen atom, when R5 represents a 2-hydroxyphenyl radical, then R3 represents a phenyl radical, or when R5 represents a 4-chlorophenyl radical, then R3 represents a 2-hydroxyphenyl radical.

10. Composition according to any one of the preceding claims, in which the derivative of formula (I) is chosen from:

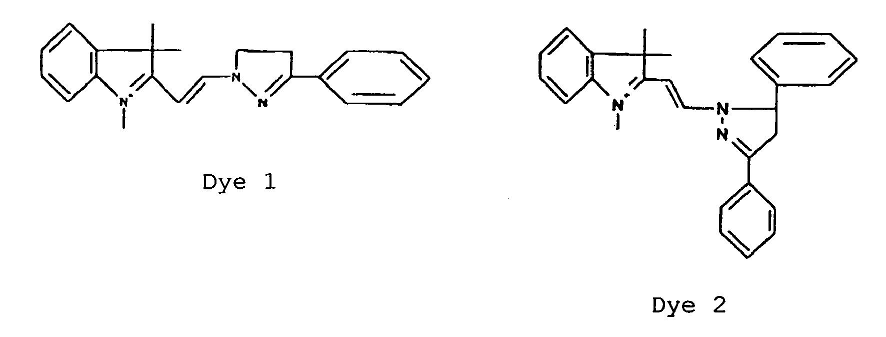

11. Composition according to any one of the preceding claims, comprising an amount of derivative of formula (I) of between 0.01% and 20% by weight relative to the total weight of the composition.

12. Composition according to any one of the preceding claims, also comprising a compound chosen from direct dyes, oxidation bases and couplers.

13. Composition according to any one of the preceding claims, comprising at least one oxidizing agent chosen from hydrogen peroxide, urea peroxide, alkali metal bromates, persalts, peracids and oxidase enzymes.

14. Composition according to any one of the preceding claims, comprising at least one additive chosen from anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrating agents, sequestering agents, fragrances, buffers, dispersants, conditioners volatile or non-volatile, modified or unmodified silicones, film-forming agents, ceramides, preserving agents and opacifiers.

15. Process for dyeing human keratin fibres, **characterized in that** it comprises the application to the keratin fibres of a composition comprising, in a suitable cosmetic medium, a 1H-pyrazolyl-ethenyl-indolium derivative of formula (I) below: 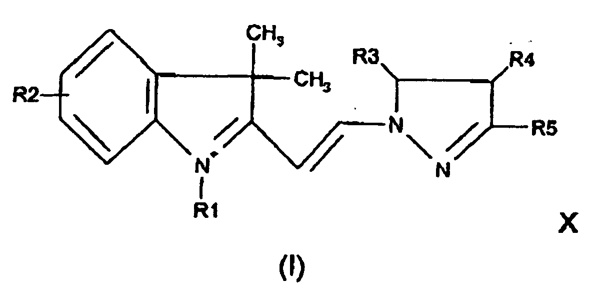 in which
● R1 represents a linear or branched C1-C4 alkyl radical or a C1-C4 aralkyl radical,
● R2 represents a hydrogen atom, a C1-C4 alkyl radical, a C1-C4 alkoxy radical, a halogen atom or a nitro group;
● R3 and R4, which may be identical or different, represent a hydrogen atom, a C1-C4 alkyl radical, an aryl radical which may be substituted with one radical R chosen from a halogen atom, a hydroxyl radical, a C1-C4 alkyl radical, a C1-C4 alkoxy radical, a carboxyl radical and a trifluoromethyl radical,
● R5 represents a hydrogen atom, a C1-C4 alkyl radical or an aryl radical that may be substituted with one radical R, and
● X is a counterion.

16. Process according to the claim 15 or 16**characterized in that** R1 represents a C1-C4 alkyl radical.

17. Process according to the claim 15 **characterized in that** R2 represents a hydrogen atom or an alkyl or alkoxy radical.

18. Process according to the claim 15 or 16 **characterized in that** R2 represents a hydrogen atom or a methyl or methoxy radical.

19. Process according to any one of the claims 15 to 18 **characterized in that** R3 and R4 represent a hydrogen atom or an alkyl or phenyl radical.

20. Process according to the claim 18 **characterized in that** R3 represents a hydrogen atom, a methyl radical or a phenyl radical, which may be substituted with one or more hydroxyl, alkoxy, methyl or chloro radicals.

21. Process according to the claim 18 **characterized in that** R4 represents a hydrogen atom or a methyl or phenyl radical, which may be substituted with one or more hydroxyl, alkoxy, methyl or chloro radicals.

22. Process according to any one of the claims 15 to 21 **characterized in that** R5 represents a hydrogen atom, an alkyl radical or a phenyl radical, which may be substituted with one or more hydroxyl, alkoxy, methyl or chloro radicals.

23. Process according to any one of the claims 15 to 22 **characterized in that** it R1 represents a methyl radical, R2 represents a hydrogen atom, R4 represents a methyl radical and, when R5 represents 4-hydroxyphenyl, then R3 represents a methyl radical, when R5 represents a 4-methoxyphenyl radical, then R3 represents a hydrogen atom, when R5 represents a 2-hydroxyphenyl radical, then R3 represents a phenyl radical, or when R5 represents a 4-chlorophenyl radical, then R3 represents a 2-hydroxyphenyl radical.

24. Process according to any one of the claims 15 to 23 **characterized in that** the derivative of formula (I) is chosen from:

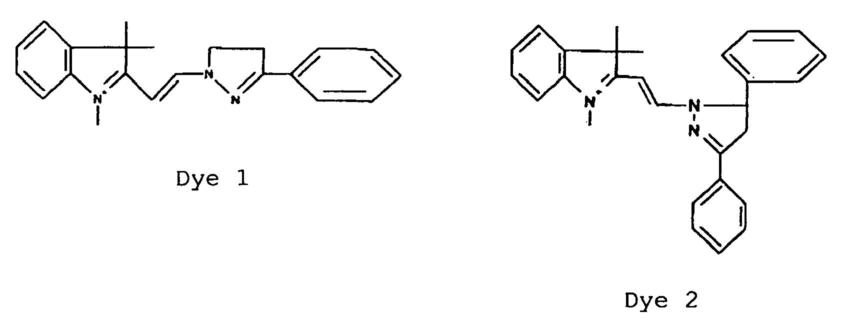

25. Process according to any one of the claims 15 to 24 **characterized in that** the composition comprises an amount of derivative of formula (I) of between 0.01% and 20% by weight relative to the total weight of the composition.

26. Process according to any one of the claims 15 to 25 **characterized in that** the composition also comprises a compound chosen from direct dyes, oxidation bases and couplers.

27. Process according to any one of the claims 15 to 26 **characterized in that** the composition comprises at least one oxidizing agent chosen from hydrogen peroxide, urea peroxide, alkali metal bromates, persalts, peracids and oxidase enzymes.

28. Process according to any one of the claims 15 to 27 **characterized in that** the composition comprises at least one anionic, cationic, nonionic, amphoteric or zwitterionic surfactant, or mixtures thereof.

29. Process according to any one of the claims 15 to 28 **characterized in that** the composition comprises at least one additive chosen from anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrating agents, sequestering agents, fragrances, buffers, dispersants, conditioners volatile or non-volatile, modified or unmodified silicones, film-forming agents, ceramides, preserving agents and opacifiers.

30. Use of a 1H-pyrazolyl-ethenyl-indolium derivative of formula (I) as defined in any one of Claims 15 to 24, for dyeing human keratin fibres.

## Patentansprüche

1. Zusammensetzung zum Färben von menschlichen Keratinfasern, die in einem geeigneten kosmetischen Medium enthält:
ein 1H-Pyrazolyl-ethenyl-indolium-Derivat der untenstehenden Formel (I) 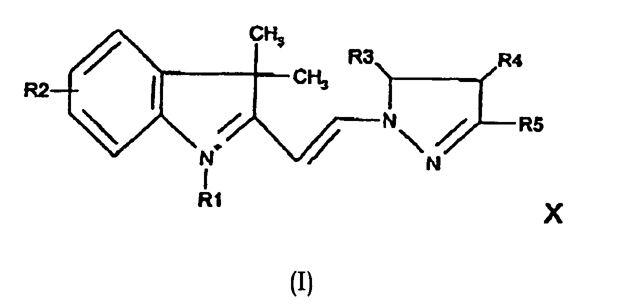
worin bedeuten:
● R1 eine geradkettige oder verzweigte C₁-C₄-Alkylgruppe oder eine C₁-C₄-Aralkylgruppe,
● R2 ein Wasserstoffatom, eine C₁-C₄-Alkylgruppe, eine C₁-C₄-Alkoxygruppe, ein Halogenatom oder eine Nitrogruppe,
● R3 und R4, die gleich oder verschieden sein können, ein Wasserstoffatom, eine C₁-C₄-Alkylgruppe, eine Arylgruppe, die mit einem Substituenten R substituiert sein kann, der ausgewählt ist unter einem Halogenatom, einer Hydroxylgruppe, einer C₁-C₄-Alkylgruppe, einer C₁-C₄-Alkoxygruppe, einer Carboxylgruppe und einer Trifluormethylgruppe,
● R5 ein Wasserstoffatom, eine C₁-C₄-Alkylgruppe oder eine Arylgruppe, die mit einem Substituenten R substituiert sein kann,
und
● X ein Gegenion,
sowie
mindestens ein anionisches, kationisches, nichtionisches, amphoteres oder zwitterionisches Tensid oder Gemische davon.

2. Zusammensetzung nach Anspruch 1, wobei R1 eine C₁-C₄-Alkylgruppe bedeutet.

3. Zusammensetzung nach Anspruch 1, bei der R2 ein Wasserstoffatom oder eine Alkylgruppe oder eine Alkoxygruppe bedeutet.

4. Zusammensetzung nach Anspruch 1 oder 2, bei der R2 ein Wasserstoffatom oder eine Methylgruppe oder eine Methoxygruppe bedeutet.

5. Zusammensetzung nach einem der vorhergehenden Ansprüche, bei der R3 und R4 ein Wasserstoffatom oder eine Alkylgruppe oder Phenylgruppe bedeuten.

6. Zusammensetzung nach Anspruch 4, bei der R3 ein Wasserstoffatom, eine Methylgruppe oder eine Phenylgruppe bedeutet, die mit einer oder mehreren Hydroxylgruppen, Alkoxygruppen, Methylgruppen oder Chlorresten substituiert sein kann.

7. Zusammensetzung nach Anspruch 4, bei der R4 ein Wasserstoffatom oder eine Methylgruppe oder eine Phenylgruppe bedeutet, die mit einer oder mehreren Hydroxylgruppen, Alkoxygruppen, Methylgruppen oder Chlorresten substituiert sein kann.

8. Zusammensetzung nach einem der vorhergehenden Ansprüche, bei der R5 ein Wasserstoffatom, eine Alkylgruppe oder eine Phenylgruppe bedeutet, die mit einer oder mehren Hydroxylgruppen, Alkoxygruppen, Methylgruppen oder Chlorresten substituiert sein kann.

9. Zusammensetzung nach einem der vorhergehenden Ansprüche, bei der R1 eine Methylgruppe, R2 ein Wasserstoffatom und R4 eine Methylgruppe bedeuten und R3 eine Methylgruppe darstellt, wenn R5 4-Hydroxyphenyl bedeutet, R3 ein Wasserstoffatom darstellt, wenn R5 eine 4-Methoxyphenylgruppe bedeutet, R3 eine Phenylgruppe darstellt, wenn R5 eine 2-Hydroxyphenylgruppe bedeutet, oder R3 eine 2-Hydroxyphenylgruppe darstellt, wenn R5 eine 4-Chlorphenylgruppe bedeutet.

10. Zusammensetzung nach einem der vorhergehenden Ansprüche, bei der das Derivat der Formel (I) ausgewählt ist unter

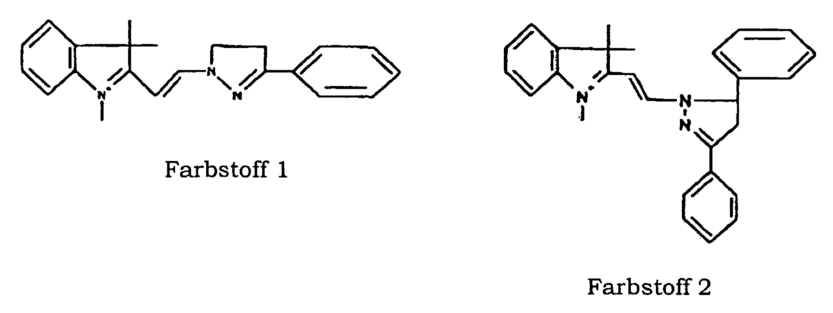

11. Zusammensetzung nach einem der vorhergehenden Ansprüche, die eine Menge des Derivats der Formel (I) von 0,01 bis 20 Gew.-%, bezogen auf das Gesamtgewicht der Zusammensetzung, enthält.

12. Zusammensetzung nach einem der vorhergehenden Ansprüche, die ferner eine Verbindung enthält, die unter Direktfarbstoffen, Oxidationsbasen und Kupplern ausgewählt ist.

13. Zusammensetzung nach einem der vorhergehenden Ansprüche, die mindestens ein Oxidationsmittel enthält, das unter Wasserstoffperoxid, Harnstoffperoxid, Alkalimetallbromaten, Persalzen, Persäuren und Oxidase-Enzymen ausgewählt ist.

14. Zusammensetzung nach einem der vorhergehenden Ansprüche, die mindestens ein Additiv enthält, das ausgewählt ist unter anionischen, kationischen, nichtionischen, amphoteren oder zwitterionischen Polymeren oder Gemischen davon, anorganischen oder organischen Verdickungsmitteln und insbesondere anionischen, kationischen, nichtionischen und amphoteren polymeren assoziativen Verdickungsmitteln, Antioxidantien, Penetrationsmitteln, Sequestriermitteln, Duftstoffen, Puffern, Dispergiermitteln, flüchtigen oder nichtflüchtigen Konditioniermitteln, modifizierten oder nichtmodifizierten Siliconen, filmbildenden Mitteln, Ceramiden, Konservierungsmitteln und Trübungsmitteln.

15. Verfahren zum Färben von menschlichen Keratinfasern, **dadurch gekennzeichnet, dass** es die Anwendung einer Zusammensetzung auf die Keratinfasern umfasst, die in einem geeigneten kosmetischen Medium ein 1H-Pyrazolyl-ethenyl-indolium-Derivat der nachstehenden Formel (I) 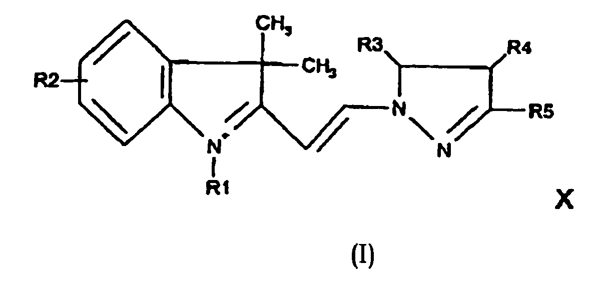 enthält, in der bedeuten:
● R1 eine geradkettige oder verzweigte C₁-C₄-Alkylgruppe oder eine C₁-C₄-Aralkylgruppe,
● R2 ein Wasserstoffatom, eine C₁-C₄-Alkylgruppe, eine C₁-C₄-Alkoxygruppe, ein Halogenatom oder eine Nitrogruppe,
● R3 und R4, die gleich oder verschieden sein können, ein Wasserstoffatom, eine C₁-C₄-Alkylgruppe, eine Arylgruppe, die mit einem Substituenten R substituiert sein kann, der ausgewählt ist unter einem Halogenatom, einer Hydroxylgruppe, einer C₁-C₄-Alkylgruppe, einer C₁-C₄-Alkoxygruppe, einer Carboxylgruppe und einer Trifluormethylgruppe,
● R5 ein Wasserstoffatom, eine C₁-C₄-Alkylgruppe oder eine Arylgruppe, die mit einem Substituenten R substituiert sein kann,
und
● X ein Gegenion.

16. Verfahren nach Anspruch 15, **dadurch gekennzeichnet, dass** R1 eine C₁-C₄-Alkylgruppe bedeutet.

17. Verfahren nach Anspruch 15, **dadurch gekennzeichnet, dass** R2 ein Wasserstoffatom oder eine Alkylgruppe oder eine Alkoxygruppe bedeutet.

18. Verfahren nach Anspruch 15 oder 16, **dadurch gekennzeichnet, dass** R2 ein Wasserstoffatom oder eine Methylgruppe oder eine Methoxygruppe bedeutet.

19. Verfahren nach einem der Ansprüche 15 bis 18, **dadurch gekennzeichnet, dass** R3 und R4 ein Wasserstoffatom oder eine Alkylgruppe oder eine Phenylgruppe bedeuten.

20. Verfahren nach Anspruch 18, **dadurch gekennzeichnet, dass** R3 ein Wasserstoffatom, eine Methylgruppe oder eine Phenylgruppe bedeutet, die mit einer oder mehreren Hydroxylgruppen, Alkoxygruppen, Methylgruppen oder Chlorresten substituiert sein kann.

21. Verfahren nach Anspruch 18, **dadurch gekennzeichnet, dass** R4 ein Wasserstoffatom oder eine Methylgruppe oder eine Phenylgruppe bedeutet, die mit einer oder mehreren Hydroxylgruppen, Alkoxygruppen, Methylgruppen oder Chlorresten substituiert sein kann.

22. Verfahren nach einem der Ansprüche 15 bis 21, **dadurch gekennzeichnet, dass** R5 ein Wasserstoffatom, eine Alkylgruppe oder eine Phenylgruppe bedeutet, die mit einer oder mehreren Hydroxylgruppen, Alkoxygruppen, Methylgruppen oder Chlorresten substituiert sein kann.

23. Verfahren nach einem der Ansprüche 15 bis 22, **dadurch gekennzeichnet, dass** R1 eine Methylgruppe, R2 ein Wasserstoffatom und R4 eine Methylgruppe bedeuten und R3 eine Methylgruppe darstellt, wenn R5 4-Hydroxyphenyl bedeutet, R3 ein Wasserstoffatom darstellt, wenn R5 eine 4-Methoxyphenylgruppe bedeutet, R3 eine Phenylgruppe darstellt, wenn R5 eine 2-Hydroxyphenylgruppe bedeutet, oder R3 eine 2-Hydroxyphenylgruppe darstellt, wenn R5 eine 4-Chlorphenylgruppe bedeutet.

24. Verfahren nach einem der Ansprüche 15 bis 23, **dadurch gekennzeichnet, dass** das Derivat der Formel (I) ausgewählt ist unter

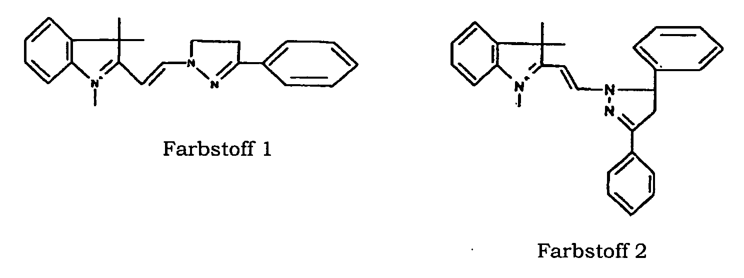

25. Verfahren nach einem der Ansprüche 15 bis 24, **dadurch gekennzeichnet, dass** die Zusammensetzung eine Menge des Derivats der Formel (I) von 0,01 bis 20 Gew.-%, bezogen auf das Gesamtgewicht der Zusammensetzung, enthält.

26. Verfahren nach einem der Ansprüche 15 bis 25, **dadurch gekennzeichnet, dass** die Zusammensetzung ferner eine Verbindung enthält, die unter Direktfarbstoffen, Oxidationsbasen und Kupplern ausgewählt ist.

27. Verfahren nach einem der Ansprüche 15 bis 26, **dadurch gekennzeichnet, dass** die Zusammensetzung mindestens ein Oxidationsmittel enthält, das unter Wasserstoffperoxid, Harnstoffperoxid, Alkalimetallbromaten, Persalzen, Persäuren und Oxidase-Enzymen ausgewählt ist.

28. Verfahren nach einem der Ansprüche 15 bis 27, **dadurch gekennzeichnet, dass** die Zusammensetzung mindestens ein anionisches, kationisches, nichtionisches, amphoteres oder zwitterionisches Tensid oder Gemische davon enthält.

29. Verfahren nach einem der Ansprüche 15 bis 28, **dadurch gekennzeichnet, dass** die Zusammensetzung mindestens ein Additiv enthält, das ausgewählt ist unter anionischen, kationischen, nichtionischen, amphoteren oder zwitterionischen Polymeren oder Gemischen davon, anorganischen oder organischen Verdickungsmitteln und insbesondere anionischen, kationischen, nichtionischen und amphoteren polymeren assoziativen Verdickungsmitteln, Antioxidantien, Penetrationsmitteln, Sequestriermitteln, Duftstoffen, Puffern, Dispergiermitteln, flüchtigen oder nichtflüchtigen Konditioniermitteln, modifizierten oder nichtmodifizierten Siliconen, filmbildenden Mitteln, Ceramiden, Konservierungsmitteln und Trübungsmitteln.

30. Verwendung eines 1H-Pyrazolyl-ethenyl-indolium-Derivats der Formel (I) wie in einem der Ansprüche 15 bis 24 definiert zum Färben von menschlichen Keratinfasern.

## Revendications

1. Composition pour la teinture des fibres kératiniques humaines comprenant, dans un milieu cosmétique approprié, un dérivé 1H-pyrazolyl-éthényl-indolium de formule (1) suivante : 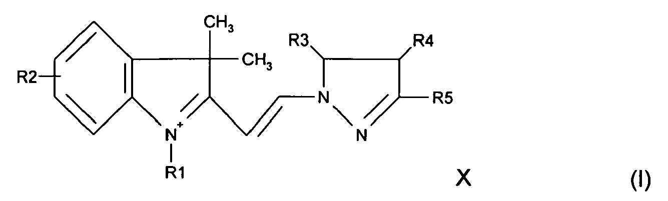 dans laquelle
● R1 représente un radical alkyle en C1-C4, linéaire ou ramifié ou un radical aralkyle en C1-C4,
● R2 représente un atome d'hydrogène, un radical alkyle en C1-C4, un radical alcoxy en C1-C4, un atome d'halogène ou un groupe nitro ;
● R3 et R4, identiques ou différents représentent un atome d'hydrogène, un radical alkyle en C1-C4 un radical aryle pouvant être substitué par un radical R choisi parmi un atome d'halogène, un radical hydroxy, un radical alkyle en C1-C4, un radical alkoxy en C1-C4, un radical carboxy, trifluorométhyle,
● R5 représente un atome d'hydrogène, un radical alkyle en C1-C4 un radical aryle pouvant être substitué par un radical R,
● X est un contre ion, et
comprenant au moins un agent tensioactif anionique, cationique, non ionique, amphotère, zwitterionique ou leurs mélanges.

2. Composition selon la revendication 1 dans laquelle R1 représente un radical alkyle en C1-C4.

3. Composition selon la revendication 1 dans laquelle R2 représente un atome d'hydrogène, un radical alkyle ou alcoxy.

4. Composition selon la revendication 1 ou 2 dans laquelle R2 représente un atome d'hydrogène, un radical méthyle ou méthoxy.

5. Composition selon l'une quelconque des revendications précédentes dans laquelle R3 et R4 représentent un atome d'hydrogène, un radical alkyle ou phényle.

6. Composition selon la revendication 4 dans laquelle R3 représente un atome d'hydrogène, un radical méthyle ou un radical phényle pouvant être substitué par un ou plusieurs radicaux hydroxy, alcoxy, méthyle ou chloro.

7. Composition selon la revendication 4 dans laquelle R4 représente un atome d'hydrogène, un radical méthyle ou phényle pouvant être substitué par un ou plusieurs radicaux hydroxy, alcoxy, méthyle ou chloro.

8. Composition selon l'une quelconque des revendications précédentes dans laquelle R5 représente un atome d'hydrogène, un radical alkyle ou un radical phényle pouvant être substitué par un ou plusieurs radicaux hydroxy, alcoxy, méthyle ou chloro.

9. Composition selon l'une quelconque des revendications précédentes dans laquelle R1 représente un radical méthyle, R2 représente un atome d'hydrogène, R4 représente un radical méthyle, et lorsque R5 représente 4-hydroxy phényle alors R3 représente un radical méthyle, lorsque R5 représente un radical 4- methoxyphényle alors R3 représente un atome d'hydrogène, lorsque R5 représente un radical 2-hydroxyphényle alors R3 représente un radical phényle ou lorsque R5 représente un radical 4-chloro phényle alors R3 représente un radical 2-hydroxyphényle.

10. Composition selon l'une quelconque des revendications précédentes dans laquelle le dérivé de formule (1) est choisi parmi

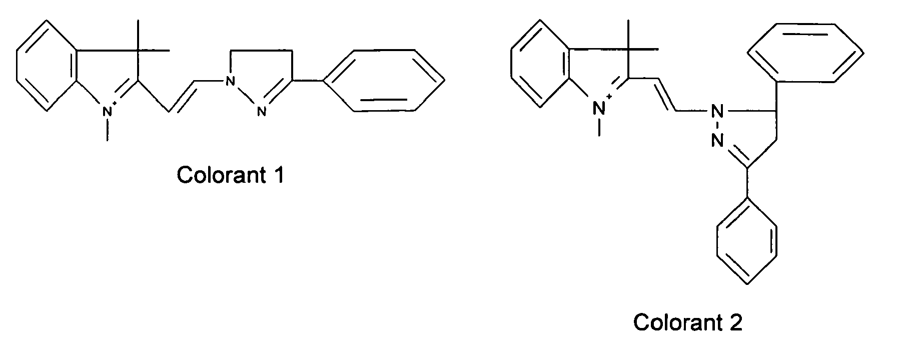

11. Composition selon l'une quelconque des revendications précédentes comprenant une quantité de dérivé de formule (I) comprise entre 0,01 et 20% en poids, par rapport au poids total de la composition.

12. Composition selon l'une quelconque des revendications précédentes comprenant de plus un composé choisi parmi les colorants directs, les bases d'oxydation, les coupleurs.

13. Composition selon l'une quelconque des revendications précédentes comprenant au moins un agent oxydant choisi parmi le peroxyde d'hydrogène, le peroxyde d'urée, les bromates de métaux alcalins, les persels, les peracides, les enzymes oxydases.

14. Composition selon l'une quelconque des revendications précédentes comprenant au moins un additif choisi parmi les polymères anioniques, cationiques, non ioniques, amphotères, zwitterioniques ou leurs mélanges, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents de conditionnement silicones volatiles ou non volatiles, modifiées ou non modifiées, les agents filmogènes, les céramides, les agents conservateurs, les agents opacifiants.

15. Procédé de teinture des fibres kératiniques humaines **caractérisé en ce qu'**il comprend l'application sur les fibres kératiniques d'une composition comprenant, dans un milieu cosmétique approprié, un dérivé 1H-pyrazolyl-éthényl-indolium de formule (1) suivante : 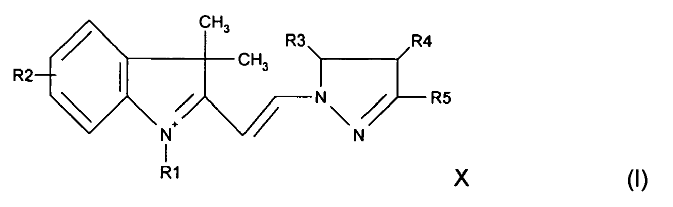 dans laquelle
● R1 représente un radical alkyle en C1-C4, linéaire ou ramifié ou un radical aralkyle en C1-C4,
● R2 représente un atome d'hydrogène, un radical alkyle en C1-C4, un radical alcoxy en C1-C4, un atome d'halogène ou un groupe nitro ;
● R3 et R4, identiques ou différents représentent un atome d'hydrogène, un radical alkyle en C1-C4 un radical aryle pouvant être substitué par un radical R choisi parmi un atome d'halogène, un radical hydroxy, un radical alkyle en C1-C4, un radical alkoxy en C1-C4, un radical carboxy, trifluorométhyle,
● R5 représente un atome d'hydrogène, un radical alkyle en C1-C4 un radical aryle pouvant être substitué par un radical R, et
● X est un contre ion.

16. Procédé selon la revendication 15 **caractérisé en ce que** R1 représente un radical alkyle en C1-C4.

17. Procédé selon la revendication 15 ou 16 **caractérisé en ce que** R2 représente un atome d'hydrogène, un radical alkyle ou alcoxy.

18. Procédé selon l'une quelconque des revendications 15 à 17 **caractérisé en ce que** R2 représente un atome d'hydrogène, un radical méthyle ou méthoxy.

19. Procédé selon l'une quelconque des revendications 15 à 18 **caractérisé en ce que** R3 et R4 représentent un atome d'hydrogène, un radical alkyle ou phényle.

20. Procédé selon la revendications 19 **caractérisé en ce que** R3 représente un atome d'hydrogène, un radical méthyle ou un radical phényle pouvant être substitué par un ou plusieurs radicaux hydroxy, alcoxy, méthyle ou chloro.

21. Procédé selon la revendication 19 **caractérisé en ce que** R4 représente un atome d'hydrogène, un radical méthyle ou phényle pouvant être substitué par un ou plusieurs radicaux hydroxy, alcoxy, méthyle ou chloro.

22. Procédé selon l'une quelconque des revendications 15 à 21 **caractérisé en ce que** R5 représente un atome d'hydrogène, un radical alkyle ou un radical phényle pouvant être substitué par un ou plusieurs radicaux hydroxy, alcoxy, méthyle ou chloro.

23. Procédé selon l'une quelconque des revendications 15 à 22 **caractérisé en ce que** R1 représente un radical méthyle, R2 représente un atome d'hydrogène, R4 représente un radical méthyle, et lorsque R5 représente 4-hydroxy phényle alors R3 représente un radical méthyle, lorsque R5 représente un radical 4-methoxyphényle alors R3 représente un atome d'hydrogène, lorsque R5 représente un radical 2-hydroxyphényle alors R3 représente un radical phényle ou lorsque R5 représente un radical 4-chloro phényle alors R3 représente un radical 2-hydroxyphényle.

24. Procédé selon l'une quelconque des revendications 15 à 23 **caractérisé en ce que** le dérivé de formule (1) est choisi parmi

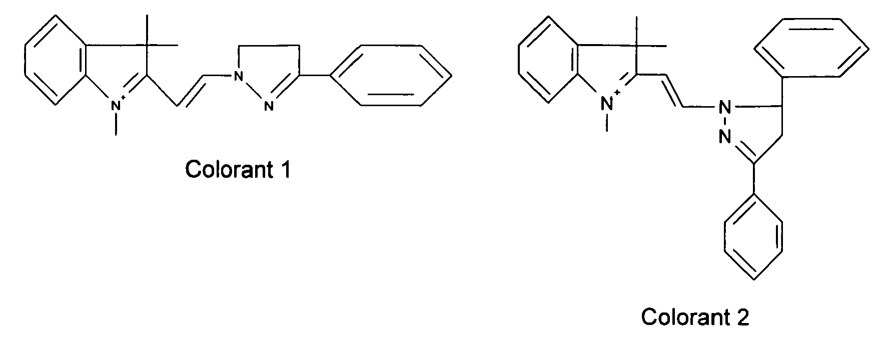

25. Procédé selon l'une quelconque des revendications 15 à 24 **caractérisé en ce que** la composition comprend une quantité de dérivé de formule (I) comprise entre 0,01 et 20% en poids, par rapport au poids total de la composition.

26. Procédé selon l'une quelconque des revendications 15 à 25 **caractérisé en ce que** la composition comprend de plus un composé choisi parmi les colorants directs, les bases d'oxydation, les coupleurs.

27. Procédé selon l'une quelconque des revendications 15 à 26 **caractérisé en ce que** la composition comprend au moins un agent oxydant choisi parmi le peroxyde d'hydrogène, le peroxyde d'urée, les bromates de métaux alcalins, les persels, les peracides, les enzymes oxydases.

28. Procédé selon l'une quelconque des revendications 15 à 27 **caractérisé en ce que** la composition comprend au moins un agent tensioactif anionique, cationique, non ionique, amphotère, zwitterionique ou leurs mélanges.

29. Procédé selon l'une quelconque des revendications 15 à 28 **caractérisé en ce que** la composition comprend au moins un additif choisi parmi les polymères anioniques, cationiques, non ioniques, amphotères, zwitterioniques ou leurs mélanges, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents de conditionnement silicones volatiles ou non volatiles, modifiées ou non modifiées, les agents filmogènes, les céramides, les agents conservateurs, les agents opacifiants.

30. Utilisation d'un dérivé 1H-pyrazolyl-éthényl-indolium de formule (I) telle que définie selon l'une quelconque des revendications 15 à 24 pour la teinture des fibres kératiniques humaines.
